# EUROPÄISCHE PATENTSCHRIFT

(11) **EP 3 936 186 B1**
(45) Veröffentlichungstag und Bekanntmachung des Hinweises auf die Patenterteilung: **29.11.2023**
(21) Anmeldenummer: 20184498.2
(22) Anmeldetag: 07.07.2020
(51) Int. Cl.: A61N 1/375, A61M 60/122, H01R 13/52, A61M 60/878, H01R 24/20, H01R 13/627, H01R 24/28

(54) **ELEKTRISCHE VERBINDUNG FÜR EINE AKTUATOREINHEIT**
ELECTRICAL CONNECTION FOR AN ACTUATOR UNIT
RACCORDEMENT ÉLECTRIQUE POUR UNE UNITÉ D'ACTIONNEUR

(43) Veröffentlichungstag der Anmeldung: 12.01.2022
(60) Teilanmeldung: 23205135.9
(73) Patentinhaber: Maxon International AG, 6072 Sachseln (CH)
(72) Erfinder: Imfeld, Matthias, 6074 Giswil (CH); Schrackmann, Remo, 6060 Sarnen (CH); Odermatt, Pius, 6383 Dallenwill (CH); Omlin, Patrik, 6060 Sarnen (CH)
(74) Vertreter: Grünecker Patent- und Rechtsanwälte PartG mbB

(56) Entgegenhaltungen:
- EP-A1- 2 787 581
- DE-A1-102018 208 539
- US-A1- 2013 190 551
- US-A1- 2015 364 861

## Beschreibung

Die Erfindung betrifft eine implantierbare Anschlusskabelbaugruppe für eine Aktuatoreinheit eines Implantats mit einem Steckverbinder und einem in dem Steckverbinder endenden Kabelstrang mit mindestens zwei Anschlusskabeln, wobei der Steckverbinder ein Steckergehäuse und mindestens zwei mit jeweils einem Anschlusskabel verbundene Kontaktelemente aufweist, die in dem Steckergehäuse fixiert sind. Darüber hinaus betrifft die Erfindung die Aktuatoreinheit eines Implantats aus einer Antriebsbaugruppe mit Elektromotor und einer solchen implantierbaren Anschlusskabelbaugruppe sowie ein Verfahren zum Herstellen einer Aktuatoreinheit eines Implantats.

In den letzten Jahren sind implantierbare medizinische Geräte, die elektrisch versorgt und betrieben werden, im Bereich der Medizin allgegenwärtig geworden. Dabei gibt es eine relativ breite Palette solcher medizinischen Geräte, von Neurostimulationsgeräten, Herzschrittmachern und Cochlea-Implantaten, Implantaten gegen Inkontinenz oder zur Appetitkontrolle bis hin zu Herzunterstützungspumpen und anderen ventrikulären Hilfsgeräten. Typischerweise erfordern solche medizinischen Geräte die Übertragung von Daten-, Energie- und/oder Steuersignalen über entsprechende Anschlusskabel und Leitungen von einer Energiequelle oder einer Steuerung zu dem implantierten Gerät. Die unterschiedlichen implantierbaren Gerätetypen haben unterschiedliche Anforderungen an die Versorgung mit elektrischer Energie oder Steuersignalen sowie unterschiedliche Anwendungs- und Einsatzbereiche, so dass Anschlusskabel und Steckverbinder üblicherweise nur für einen Gerätetyp geeignet sind. Während Neurostimulationsgeräte eine implantierte Anschlussleitung benötigen, die elektrisch mit einem Kopf eines implantierten Impulsgenerators verbunden ist, benötigen insbesondere ventrikuläre Hilfsgeräte und andere Herzunterstützungssysteme eine elektrische Energieversorgung mit relativ hohen Strom- und Spannungsanforderungen. Entsprechend sind Verbindungssysteme und Anschlusskabel für implantierte Geräte üblicherweise nicht für andere implantierte medizinische Geräte verwendbar.

Im Vergleich zu Herzschrittmachern, die typischerweise niedrige oder intermittierende Leistungsanforderungen haben, weisen ventrikuläre Hilfsgeräte wie Herzunterstützungspumpen eine erhöhte Leistungsanforderung mit hohen Stromstärken und hohen Dauerspannungen auf. Da ein Stromausfall in einer Herzunterstützungspumpe lebensbedrohliche Folgen haben kann, müssen sowohl der Steckverbinder als auch der damit verbundene Kabelstrang über einen langen Zeitraum eine zuverlässige elektrische Verbindung gewährleisten, um einen kontinuierlichen Betrieb des implantierten Geräts zu gewährleisten. Da das Implantieren von elektrischen Steckverbindern für höhere Leistungsanforderungen aufgrund der zyklischen Belastungen durch die Biegung und Bewegung des Kabelstrangs, des Steckverbinders und der Aktuatoreinheit im Körper mit einem hohen Risiko verbunden ist, werden derartige Geräte über einen Kabelstrang mit Strom versorgt, der direkt an den Motor der implantierten Herzunterstützungspumpe angeschlossen ist. Der Anschluss des Kabelstrangs an eine Stromversorgung kann dann außerhalb des Körpers oder zumindest an einem vom Herz entfernten Ort erfolgen, der leichter zugänglich ist, und stabiler gehalten werden kann.

Eine weitere Herausforderung bei implantierten Steckverbindern wie insbesondere bei Inkontinenz-Okklusionssystemen oder bei im Magen oder Verdauungstrakt angeordneten Appetitkontrollsystemen besteht darin, dass die mit Flüssigkeit gefüllte Umgebung im menschlichen Körper korrosiv gegenüber den im Steckverbinder eingesetzten Materialien sein kann, beispielsweise gegenüber Kupfer und Edelstahl, die herkömmlicherweise in Hochleistungs-Steckverbindern verwendet werden. Demgegenüber weisen nicht-korrosive Metalllegierungen, beispielsweise eine Platin-Iridium-Legierung, problematische mechanische Eigenschaften, beispielsweise eine hohe Sprödigkeit auf, die die Verarbeitung und den Einsatz in einer implantierten Aktuatoreinheit sowie den Anschluss eines Kabelstrangs schwierig gestalten. Darüber hinaus sind derartige nicht-korrosive Metalllegierungen außerordentlich teuer.

Aus der Druckschrift US 2015/364861 A1 ist beispielsweise ein implantierbares Verbindungssystem zur kontinuierlichen Versorgung eines Implantats mit einer hohen elektrischen Leistung bekannt, bei der mehrere mit einzelnen Anschlusskabeln versehene Steckverbinder mit einzelnen Buchsen verbunden sind, die wiederum über entsprechende Anschlusskabel mit der Aktuatoreinheit des Implantats verbunden sind. Dabei sichert ein Rückhaltemechanismus eine mechanische und elektrische Verbindung zwischen dem Steckverbinder und der Buchsenaufnahme.

Weiterhin ist aus der Druckschrift US 2013/190 551 A1 eine implantierbare Blutpumpe mit einer Kabelbaugruppe bekannt bei der ein Kabelstrang über eine Zugentlastung an der Blutpumpe befestigt ist, um die Blutpumpe mit Energie und Steuersignalen zu versorgen. Dazu sind die einzelnen Leiter des Kabelstrangs mit zugehörigen Kabelaufnahmen verbunden, wobei die Kabelaufnahmen auf die Kontakte der Blutpumpe aufgesteckt werden und optional mit diesen laserverschweißt werden können. Die einzelnen Leiter werden innerhalb einer Anschlusskammer von einem Träger gehalten, die nach der Verbindung der Leiter mit den Kontakten durch eine Epoxidharz-Gussmasse gefüllt werden kann.

Im Stand der Technik werden verschiedene Konzepte für implantierbare Steckverbinder oder Kabelanschlüsse vorgeschlagen oder theoretisch beschrieben, jedoch sind angesichts der konstruktiven Herausforderungen, die mit implantierbaren Vorrichtungen verbunden sind, viele davon übermäßig sperrig, teuer und zu fehleranfällig. Es besteht daher ein Bedarf für eine implantierbare Anschlusskabelbaugruppe mit einem Steckverbinder und einem in dem Steckverbinder endenden Kabelstrang, der sowohl mit den erhöhten Leistungs- und Materialanforderungen von ventrikulären Herzunterstützungssystemen, Inkontinenz-Okklusionssystemen oder Appetitkontrollsystemen verwendbar ist, sicher einsetzbar und korrosionsbeständig ist sowie gute mechanische Eigenschaften bei reduzierten Material- und Herstellungskosten aufweist. Weiterhin ist es wünschenswert, dass ein solcher Steckverbinder entsprechend kompakt realisierbar ist, um zusammen mit einem Implantat an verschiedenen Stellen innerhalb des Körpers implantiert werden zu können.

Es ist daher die Aufgabe der vorliegenden Erfindung, eine implantierbare Anschlusskabelbaugruppe der eingangs genannten Art bereitzustellen, die eine sichere elektrische und mechanische Verbindung mit einer Antriebsbaugruppe der Aktuatoreinheit eines Implantats ermöglicht, kostengünstig herstellbar ist und problemlos implantierbar ist.

Diese Aufgabe wird durch eine implantierbare Anschlusskabelbaugruppe gemäß Anspruch 1 sowie ein Verfahren zum Herstellen einer Aktuatoreinheit eines Implantats mit einer implantierbaren Anschlusskabelbaugruppe gemäß Anspruch 10 gelöst.

Bei dem Implantat handelt es sich insbesondere um ein Herzunterstützungssystem, ein Inkontinenz-Okklusionssystem oder ein Appetitkontrollsystem. Das Appetitkontrollsystem ist vorzugsweise im Magen oder Darmtrakt angeordnet.

Die erfindungsgemäße implantierbare Anschlusskabelbaugruppe kann sehr einfach mit einer Antriebsbaugruppe verbunden werden, um eine elektrische Verbindung für eine leistungsfähige und sichere Spannungsversorgung der Aktuatoreinheit zu ermöglichen. Dabei ist die Anschlusskabelbaugruppe mit dem Steckverbinder und dem im Steckverbinder endenden Kabelstrang in der Montage der Aktuatoreinheit lösbar zu der Antriebsbaugruppe positioniert und wird erst in einem abschließenden Verfahrensschritt unlösbar zu einer Aktuatoreinheit verbunden, um als Teil des implantierbaren medizinischen Geräts in einem Körper implantiert werden zu können. So können sowohl die Anschlusskabelbaugruppe als auch eine Antriebsbaugruppe mit Elektromotor der Aktuatoreinheit separat hergestellt werden, wobei insbesondere bei der Herstellung der Antriebsbaugruppe keine Anschlusskabel und Kontakte berücksichtigt werden müssen, sodass die elektrischen Verbindungsstellen keinen mechanischen Belastungsrisiken ausgesetzt sind. Im Gegensatz zu der herkömmlichen Produktion von implantierbaren medizinischen Geräten, können diverse Fertigungsschritte außerhalb eines Reinraums durchgeführt werden und die einzelnen Baugruppen können vor ihrer Verbindung in der Endmontage auf ihre Dichtheit gegenüber Flüssigkeiten überprüft und die elektrische Verbindung getestet werden. Bei festgestellten Fehlern kann sowohl die implantierbare Anschlusskabelbaugruppe als auch eine Antriebsbaugruppe der Aktuatoreinheit vor der Endmontage ausgetauscht und ersetzt werden. Neben dem geringen Ausschuss durch Vermeidung von Herstellungsrisiken reduzieren sich so auch die Kosten für den verbleibenden Ausschuss. Der Kabelstrang umfasst mindestens zwei Anschlusskabel, bevorzugt mindestens drei Anschlusskabel, die an ihren Enden jeweils einer Kabelaufnahme der mindestens zwei Kontaktelemente, bevorzugt mindestens drei Kontaktelemente, zugeordnet und für eine gute elektrische Verbindung mit dieser verlötet sein können. Die Kabelaufnahme kann eine Kabelbuchse zur Kontaktierung der aus dem Ende des Kabelstrangs austretenden Anschlusskabel aufweisen. Die Kontaktelemente bestehen direkt aus einem elektrisch gut leitfähigen Werkstoff oder sind mit einer gut leitfähigen Beschichtung versehen. Das Steckergehäuse des Steckverbinders weißt eine Kabelöffnung zur Durchführung des Kabelstrangs in den Steckverbinder auf, wobei der Kabelmantel des Kabelstrangs, der bevorzugt aus Silikon oder aus einem thermoplastischen Elastomer auf Urethanbasis besteht (TPU) besteht, erst innerhalb des Steckergehäuses die mindestens zwei Anschlusskabel freigibt und eine Verbindung der Anschlusskabel mit den Kontaktelementen ermöglicht. Dabei werden die Hohlräume, die sich innerhalb des Steckergehäuses zwischen der Kabelöffnung für den Kabelstrang und den zur Antriebseinheit hin offenliegenden Kontaktelementen ergeben, mit einer Vergussmasse vergossen, die das durch die Kabelöffnung im Steckergehäuse eingeführte Ende des Kabelstrangs und die mindestens zwei Anschlusskabel hermetisch dicht umschließt.

Eine besondere Ausführungsform sieht vor, dass eine elektrisch isolierende Aufnahme vorgesehen ist, in der die Kontaktelemente fixiert sind, wobei die elektrisch isolierende Aufnahme in dem Steckergehäuse aufgenommen ist. Die elektrisch isolierende Aufnahme ermöglicht eine sichere Positionierung der Kontaktelemente relativ zum Steckergehäuse und eine gute Abgrenzung gegenüber den vergossenen Hohlräumen, die sich im Inneren des Steckergehäuses zwischen der elektrisch isolierenden Aufnahme mit den Kontaktelementen und der Kabelöffnung für den Kabelstrang ergeben. Dabei können die Kontaktelemente durch Einkleben, Einpressen, thermisches Fügen oder einer Kombination unterschiedlicher Fügeverfahren in der elektrisch isolierenden Aufnahme verankert sein.

Eine weitere Ausbildung sieht vor, dass die elektrisch isolierende Aufnahme eine Bohrung aufweist, bevorzugt eine im Wesentlichen mittig angeordnete Bohrung, wobei die Vergussmasse zum Vergießen der Hohlräume durch die Bohrung in das Steckergehäuse eingebracht ist. Bei der Montage der Anschlusskabelbaugruppe wird die elektrisch isolierende Aufnahme in einer Anschlussöffnung des Steckergehäuses positioniert und zur Abdichtung und Fixierung der mindestens zwei Anschlusskabel, sowie des Endes des Kabelstrangs in der Kabelöffnung mit einer Vergussmasse vergossen, um die elektrisch isolierende Aufnahme mit den mindestens zwei Kontaktelementen im Steckergehäuse zu fixieren und die Anschlusskabel und das Ende des Kabelstrangs abzudichten. Dabei erleichtert eine mittige Bohrung in der elektrisch isolierenden Aufnahme das Vergießen der Hohlräume mit der Vergussmasse. Die Bohrung ist eine Durchgangsbohrung zwischen der zur Umgebung hin offenen Seite der elektrisch isolierenden Aufnahme und den Hohlräumen im Steckergehäuse. Zur Reduzierung der Oberfläche des Steckverbinders kann nach dem Vergießen der Hohlräume des Steckergehäuses mit der Vergussmasse auch die Bohrung der elektrisch isolierenden Aufnahme mit Vergussmasse gefüllt sein. Die mittige Bohrung in der elektrisch isolierenden Aufnahme erleichtert die Herstellung des Steckverbinders und das Vergießen der Hohlräume mit der Vergussmasse.

Vorteilhafterweise kann die Vergussmasse eine biokompatible Silikon-Vergussmasse und/oder eine biokompatible Epoxidharz-Vergussmasse sein, wobei die Vergussmasse bevorzugt aus mindestens zwei Bereichen unterschiedlicher Viskosität besteht, wobei mindestens ein Bereich aus einer dünnflüssigen Vergussmasse und mindestens ein Bereich aus einer zähflüssigen Vergussmasse besteht. Sowohl die biokompatible Silikon-Vergussmasse, als auch die biokompatible Epoxidharz-Vergussmasse ermöglichen eine untoxische, nicht reaktive und gas- und flüssigkeitsundurchlässige Abdichtung des Steckverbinders und des im Steckergehäuse endenden Kabelstrangs. Dabei kann für eine sichere Fixierung der Anschlusskabel in dem Hohlraum des Steckergehäuses und elastischen Anschluss des Kabelstrangs an das Steckergehäuse die Vergussmasse in mindestens einem Bereich, bevorzugt im Bereich der Kabelöffnung, aus biokompatibler Silikon-Vergussmasse und in mindestens einem Bereich aus biokompatibler Epoxidharz-Vergussmasse bestehen.

Für eine einfache erfindungsgemäße Ausbildung der separaten Verbindungsschnittstellen der mindestens zwei Kontaktelemente zur elektrischen Verbindung mit der Antriebsbaugruppe der Aktuatoreinheit, sowie für einen flachen Aufbau der Kontaktelemente, sind die Verbindungsschnittstellen der mindestens zwei Kontaktelemente als Kontaktbuchsen zur Aufnahme von Kontaktstiften ausgebildet. Diese Kontaktbuchsen können bei der Montage der Anschlusskabelbaugruppe mit der Antriebsbaugruppe der Aktuatoreinheit entsprechende Kontaktstifte sicher aufnehmen, die an einem Motorflansch der Antriebsvorgruppe vorstehen können.

Um für eine erfindungsgemäße Aktuatoreinheit eine gute elektrische Verbindung der Kontaktbuchsen mit den Kontaktstiften einer Antriebsbaugruppe zu ermöglichen, sowie für einen sicheren Halt und eine geringe Bewegungsfreiheit der Kontaktstifte in den Kontaktbuchsen zu realisieren, weisen die Kontaktbuchsen zur Aufnahme von Kontaktstiften eine Nut auf, in der eine elektrisch leitende, schräg gewickelte Feder angeordnet ist, die einen elektrischen Kontakt mit den Kontaktstiften ausbildet. Eine solche schräg gewickelte Feder ermöglicht vorteilhafterweise eine redundante, mehrfache Kontaktierung der Kontaktstifte in der Kontaktbuchse. Alternativ können auch biegsame Federelemente zur Kontaktierung der Kontaktstifte in den als Kontaktbuchsen ausgebildeten Verbindungsschnittstellen genutzt werden.

Günstigerweise können die Kontaktelemente für eine optimierte Stromübertragung vergoldet oder aus einer korrosionsbeständigen Metalllegierung ausgebildet sein, insbesondere einer Legierung mit Platin und/oder Iridium, auch um in einem implantierten Zustand der Anschlusskabelbaugruppe eine Reaktion mit Körperflüssigkeiten zu vermeiden und eine toxische Belastung des Patienten zu verhindern.

Eine sinnvolle Ausgestaltung sieht vor, dass das Steckergehäuse aus einem schweißbaren, biokompatiblen Metall ausgebildet ist, bevorzugt aus einem korrosionsbeständigen, schweißbaren Metall. Vorzugsweise ist das Steckergehäuse aus einer Titanlegierung, insbesondere aus TiAI6V4 ausgebildet. Alternativ kann das Steckergehäuse auch einem schweißbaren, bio-kompatiblen Kunststoff ausgebildet sein. Dies ermöglicht bei der Endmontage der Anschlusskabelbaugruppe eine unlösbare Schweißverbindung mit der Antriebsbaugruppe eines Implantats, wodurch die beiden Baugruppen dicht und dauerhaft miteinander verbunden sind. So können die Anschlusskabelbaugruppe und die Antriebsbaugruppe der Aktuatoreinheit, die einzeln hermetisch abgedichtet sind und ein Eindringen von Körperflüssigkeiten verhindern, auch als gesamte Aktuatoreinheiten nach Außen hermetisch dicht miteinander verbunden sein.

Eine alternative Ausführung sieht vor, dass in dem Hohlraum zwischen dem Kabelstrang und dem Steckergehäuse ein Erdungskontakt vorgesehen ist, bevorzugt ein schräg gewickelter Federkontakt. Ein solcher Erdungskontakt ermöglicht eine elektrisch leitende Verbindung zwischen dem Kabelschirm des Kabelstrangs und dem Steckergehäuse, wobei der Kabelmantel am Ende des Kabelstrangs partiell entfernt ist und ein Erdungskontakt mit dem Steckergehäuse hergestellt ist, bevorzugt mittels einer schräg gewickelten Ringfeder, die sowohl auf dem Kabelschirm aufliegt, als auch gegen das Steckergehäuse gedrückt wird. Dies ermöglicht eine einfache und sichere elektrische Verbindung zwischen dem Kabelschirm des Kabelstrangs und dem Steckergehäuse und vermeidet so das Vorsehen eines zusätzlichen Erdungsanschlusses. Alternativ kann auch ein zusätzliches viertes Anschlusskabel als Erdungsanschluss vorgesehen sein.

Neben der Energieversorgung können auch zusätzliche Verbindungen zur Datenübertragung von Sensorsignalen vorgesehen werden. Da der Bauraum für Implantate üblicherweise so klein wie möglich gehalten werden muss, wäre eine sekundäre Nutzung der Kabelstränge, die primär der Energieversorgung/Stromübertragung dienen, durch überlagerte Übertragung von Sensorsignalen denkbar, wie sie beispielsweise aus der "Einkabeltechnologie" (OCT) bekannt ist. Neben dem kompakteren Bauraum erhöht eine verringerte Anzahl an elektrischen Kontaktierungen die Gesamtzuverlässigkeit der elektrischen Verbindungen.

Des Weiteren kann die implantierbare Anschlusskabelbaugruppe mit einer Antriebsbaugruppe mit Elektromotor zu einer Aktuatoreinheit eines Implantats verbindbar sein, wodurch neben einer einfachen Montage der Anschlusskabelbaugruppe und der Antriebsbaugruppe auch eine sichere elektrische Verbindung zwischen den beiden Komponenten der Aktuatoreinheit eines Implantats möglich ist.

Des Weiteren betrifft die vorliegende Erfindung eine Aktuatoreinheit eines Implantats, aus einer separat hergestellten Antriebsbaugruppe mit Elektromotor und einer separat hergestellten implantierbaren Anschlusskabelbaugruppe nach einer der zuvor beschriebenen Ausführungsformen, wobei die Antriebsbaugruppe und die implantierbare Anschlusskabelbaugruppe unlösbar miteinander verbunden sind, insbesondere glatt und übergangslos laserverschweißt sind. Hierzu wird insbesondere ein Continuous-wave (CW) Laserschweißverfahren angewendet. Die Kontaktierung der beiden Baugruppen erfolgt durch das Verbinden der komplementär ausgebildeten Kontaktelemente der Baugruppen, üblicherweise durch das Einschieben von Kontaktstiften in zugehörige Kontaktbuchsen, wobei die Antriebsbaugruppe und die implantierbare Anschlusskabelbaugruppe während der Montage lösbar miteinander gekoppelt sind und erst in dem abschließenden Herstellungsschritt der Aktuatoreinheit unlösbar miteinander verbunden werden. Zweckmäßigerweise werden hierzu die Gehäuse der beiden Baugruppen mittels eines Laserschweißverfahrens miteinander verschweißt. So können diverse Fertigungsschritte für die separat hergestellten Baugruppen in normalen Umgebungsbereichen durchgeführt werden, die Baugruppen separat getestet und gereinigt werden und lediglich die notwendigen Fertigungsschritte der Endmontage unter entsprechenden Reinheitsbedingungen stattfinden. Bei dem Implantat handelt es sich insbesondere um ein Herzunterstützungssystem, ein Inkontinenz-Okklusionssystem oder ein Appetitkontrollsystem. Das Appetitkontrollsystem ist vorzugsweise im Magen oder Darmtrakt angeordnet.

Darüber hinaus erstreckt sich die vorliegende Erfindung auch auf ein Verfahren zum Herstellen einer Aktuatoreinheit eines Implantats mit einer Antriebsbaugruppe mit Elektromotor und einer implantierbaren Anschlusskabelbaugruppe, das Verfahren umfasst das separate Herstellen der Antriebsbaugruppe, wobei die Antriebsbaugruppe elektrische Kontakte zum elektrischen Verbinden mit der Anschlusskabelbaugruppe aufweist; das separate Herstellen der implantierbaren Anschlusskabelbaugruppe mit einem Steckverbinder und einem mit dem Steckverbinder endenden Kabelstrang, wobei der Steckverbinder Kontaktelemente zum elektrischen Verbinden mit den elektrischen Kontakten der Antriebsbaugruppe aufweist; das lösbare Verbinden der Antriebsbaugruppe und der implantierbaren Anschlusskabelbaugruppe während der Montage der Aktuatoreinheit; und das anschließende unlösbare Verbinden der Antriebsbaugruppe und der implantierbaren Anschlusskabelbaugruppe. Das lösbare Verbinden der Antriebsbaugruppe und der implantierbaren Anschlusskabelbaugruppe während der Montage der Aktuatoreinheit, ermöglicht ein separates Herstellen der Baugruppen, wodurch die elektrischen Verbindungsstellen der Antriebsbaugruppe und der Anschlusskabelbaugruppe weniger mechanischen Belastungsrisiken ausgesetzt sind, da bei der Herstellung der Antriebsbaugruppe keine Anschlusskabel beachtet werden müssen. Des Weiteren lassen sich vor dem unlösbaren Verbinden der Antriebsbaugruppe mit der implantierbaren Anschlusskabelbaugruppe elektrische Verbindungsmessungen und mechanische Tests vornehmen, sodass und nur einwandfreie Baugruppen dem anschließenden Herstellungsschritt der Aktuatoreinheit zugeführt werden, um den Ausschuss und die Kosten der aussortierten Komponenten zu reduzieren. Bei dem Implantat handelt es sich insbesondere um ein Herzunterstützungssystem, ein Inkontinenz-Okklusionssystem oder ein Appetitkontrollsystem. Das Appetitkontrollsystem ist vorzugsweise im Magen oder Darmtrakt angeordnet.

Eine günstige Variante des Verfahrens zum Herstellen einer Aktuatoreinheit sieht vor, dass die separat hergestellte Antriebsbaugruppe und die separat hergestellte, implantierbare Anschlusskabelbaugruppe hermetisch gegen Flüssigkeiten und Gase abgedichtet sind. Das unlösbare Verbinden der Baugruppen im anschließenden Herstellungsschritt der Aktuatoreinheit ermöglicht in Kombination mit den bereits hermetisch gegen Flüssigkeiten und Gase abgedichteten Antriebs- und Anschlusskabelbaugruppen einen erhöhten Schutz der Aktuatoreinheit gegen das Eindringen von Flüssigkeiten und Gase über die zweifache hermetische Abdichtung. Das anschließende unlösbare Verbinden erfolgt vorzugsweise mittels Schweißen und insbesondere mittels Continuous-wave Laserschweißen.

Des Weiteren kann die separate Herstellung der implantierbaren Anschlusskabelbaugruppe das Vergießen der Hohlräume des Steckverbinders zwischen einem Steckergehäuse, dem Kabelstrang, den aus dem Kabelstrang austretenden Anschlusskabeln und den mit den Anschlusskabeln verbundenen Kontaktelementen des Steckverbinders mit einer Vergussmasse, insbesondere mit einer biokompatiblen Silikon-Vergussmasse und/oder einer biokompatiblen Epoxidharz-Vergussmasse umfassen. Das Vergießen der vorhandenen Hohlräume im Steckergehäuse des Steckverbinders mittels einer Vergussmasse ermöglicht eine gute Abdichtung und Fixierung des Kabelstrangs, sowie die Vermeidung jeglicher Lufteinschlüsse. Vorteilhafterweise wird hierfür eine biokompatible Silikon-Vergussmasse und/oder eine biokompatible Epoxidharz-Vergussmasse verwendet, die eine untoxische, biokompatible, nicht reaktive und gas- und flüssigkeitsundurchlässige Abdichtung des Steckverbinders und des im Steckergehäuse endenden Kabelstrangs ermöglichen. Dabei kann zur sicheren Fixierung der Anschlusskabel in dem Hohlraum des Steckergehäuses und für einen elastischen Anschluss des Kabelstrangs an das Steckergehäuse die Vergussmasse aus mindestens zwei Bereichen unterschiedlicher Viskosität bestehen, wobei ein Bereich aus einer dünnflüssigen Vergussmasse und mindestens ein Bereich aus einer zähflüssigen Vergussmasse besteht.

Eine sinnvolle Verfahrensvariante sieht vor, dass das unlösbare Verbinden der Antriebsbaugruppe und der implantierbaren Anschlusskabelbaugruppe in einem abschließenden Herstellungsschritt der Aktuatoreinheit insbesondere mittels Schweißen und vorzugsweise mittels Continuous-wave Laserschweißen erfolgt. Das Verschweißen der separat hergestellten Antriebsbaugruppe mit der separat hergestellten implantierbaren Anschlusskabelbaugruppe ermöglicht im anschließenden Herstellungsschritt der Aktuatoreinheit eine dichte und dauerhafte Verbindung der Baugruppen, sowie eine nach Außen hermetisch abgedichtete Aktuatoreinheit, so dass ein Eindringen von Körperflüssigkeiten in die Aktuatoreinheit des Implantats sicher verhindert wird.

Im Folgenden wird eine besondere Ausführungsform der Verbindung anhand beispielhafter Zeichnungen näher erläutert. Es zeigen:
Fig. 1 Eine geschnittene Seitenansicht einer Antriebsbaugruppe und einer erfindungsgemäßen implantierbaren Anschlusskabelbaugruppe einer Aktuatoreinheit eines Implantats,
Fig. 2 eine geschnittene Seitenansicht der unlösbar verbundenen Aktuatoreinheit aus Fig. 1,
Fig. 3 eine perspektivische Explosionsdarstellung der erfindungsgemäßen Anschlusskabelbaugruppe,
Fig. 4 eine teilweise geschnittene perspektivische Darstellung der unverbundenen Aktuatoreinheit aus Fig. 1,
Fig. 5 eine geschnittene Detailansicht einer weiteren Ausführungsform der implantierbaren Anschlusskabelbaugruppe für eine Aktuatoreinheit nach Fig. 2.

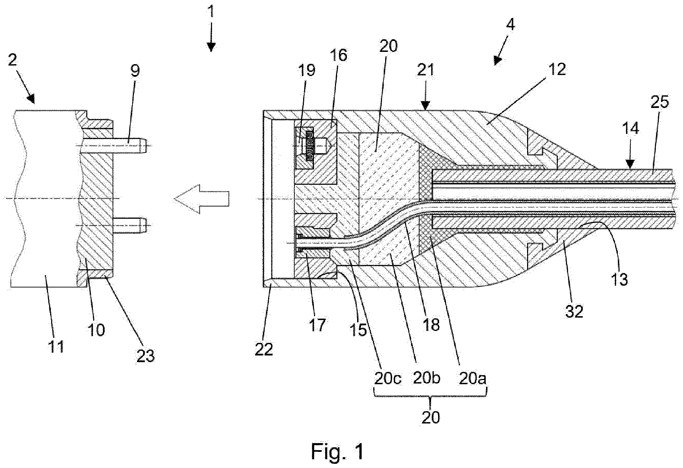

Die in Fig. 1 dargestellte Aktuatoreinheit 1 eines Implantats weist eine Antriebsbaugruppe 2 mit einem Elektromotor, sowie eine erfindungsgemäße implantierbare Anschlusskabelbaugruppe 4 auf. Der Elektromotor (nicht dargestellt) der Antriebsbaugruppe 2 weist vorzugsweise neben der Motorwelle, die von einem Motorlager gelagert ist, auch eine Motorleiterplatte zur Steuerung und Spannungsversorgung der Wicklungen des Elektromotors auf, wobei in der Mitte der Motorleiterplatte ein Zapfen zur Führung der Motorwelle vorgesehen ist. Der Elektromotor ist mit einer Statorwicklung versehen, die bevorzugt elektrisch mit der Motorleiterplatte verbunden und über die Motorleiterplatte verschaltet ist. Die Statorwicklungen werden über Kontaktstifte 9, die ebenfalls mit der Motorleiterplatte elektrisch verbunden sind, elektrisch versorgt. Die Kontaktstifte 9 können fest auf einer Motorleiterplatte angeordnet sein. Die freien Enden der Kontaktstifte 9 ragen axial aus dem mit einer Vergussmasse 10, insbesondere einer Epoxidharz-Vergussmasse, hermetisch abgedichteten Motorgehäuse 11 der Antriebsbaugruppe 2 vor und ermöglichen eine elektrische Verbindung der Motorleiterplatte mit der Anschlusskabelbaugruppe 4. Alternativ könnte hier auch eine Mehrfachkontaktdurchführung (multipin feedthrough), welche am Umfang oder stirnseitig aus dem gleichen Material wie das Motorgehäuse besteht und mit diesem verschweißt ist, verwendet werden. Als dichte Isolation könnte hierbei ein keramischer Werkstoff dienen. Die Verwendung einer Motorleiterplatte wäre bei dieser alternativen Ausführung nicht zwingend erforderlich.

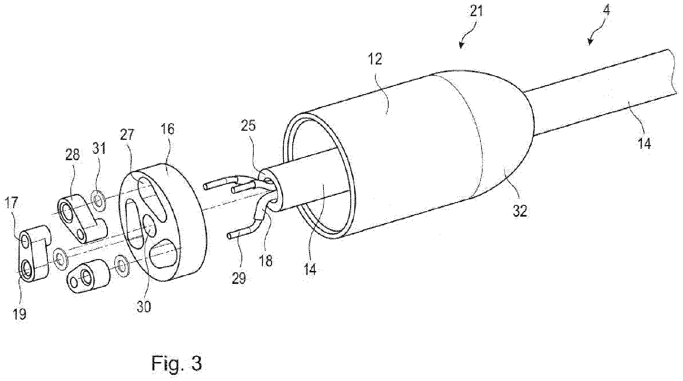

Die Anschlusskabelbaugruppe 4 umfasst ein Steckergehäuse 12 in dem durch eine Kabelöffnung 13 das Ende eines Kabelstrangs 14 hineinragt und eine in der gegenüberliegenden Anschlussöffnung 15 angeordnete elektrisch isolierende Aufnahme 16 mit entsprechenden Kabelaufnahmen. Die Kabelaufnahmen weisen Kabelbuchsen 17 zur Kontaktierung der aus dem Ende des Kabelstrangs 14 austretenden Anschlusskabel 18 auf. An den Anschlusskabeln können optional Crimp-Hülsen angebracht sein. Ferner sind in der isolierenden Aufnahme 16 Kontaktbuchsen 19 aufgenommen, die einen elektrischen Kontakt mit den Enden der Kontaktstifte 9 der Antriebsbaugruppe 2 ermöglichen. Die Kabelbuchsen 17 und die Kontaktbuchsen 19 sind vorzugsweise Bestandteil eines einteilig ausgeführten elektrischen Kontaktelements 28 wie in Fig.3 dargestellt. Die Hohlräume in dem Steckergehäuse 12, zwischen dem Ende des Kabelstrangs 14 und der elektrisch isolierenden Aufnahme 16, sind mit einer Vergussmasse 20 vergossen, um den Steckverbinder 21 der Anschlusskabelbaugruppe 4 hermetisch abzudichten, sodass keine Reinigungs-, Spül- oder Körperflüssigkeiten, sowie keine unerwünschten Gase, wie sie beispielsweise bei einer Ethylenoxid-Sterilisation (EtO-Sterilisation) auftreten, eindringen können, die dann später mit schädlicher Wirkung verzögert austreten und in die Anschlusskabelbaugruppe 4 eindringen können. Die Vergussmasse 20 besteht bevorzugt aus drei Vergussmassenschichten 20a, 20b und 20c. Die Vergussmassenschichten 20a und/oder 20b sind hierbei bevorzugt mit einer zähflüssigen Vergussmasse ausgeführt, damit sie bei der Montage der Anschlusskabelbaugruppe 4 nicht "auslaufen", während die Vergussmassenschicht 20c bevorzugt mit einer niederviskosen, dünnflüssigen Vergussmasse ausgeführt ist. Mittels der Vergussmassenschicht 20c können Hohlräume zwischen den verdrillten Einzelleitern der Kabelenden und der Isolation abgedichtet werden. Dazu ist es wichtig, dass die blanken Kabelenden der Anschlusskabel 18 sich vollständig in der Vergussmassenschicht 20c befinden. In der Anschlussöffnung 15 des Steckergehäuses 12 ist zwischen der elektrisch isolierenden Aufnahme 16 und dem Ende des Steckergehäuses 12 ein Anschlussflansch 22 vorgesehen, der zu einer entsprechenden Anschlussstufe 23 in dem Motorgehäuse 11 zusammenpasst, um ein elektrisches Kontaktieren der an dem Ende der Anschlussstufe 23 aus dem Motorgehäuse 11 hervorstehenden Kontaktstifte 9 mit den Kontaktbuchsen 19 der Anschlusskabelbaugruppe 4 zu ermöglichen. Dies ermöglicht während der Montage der Aktuatoreinheit 1 eine lösbare elektrische Verbindung zwischen der Antriebsbaugruppe 2 und der Anschlusskabelbaugruppe 4, sodass neben den einzelnen Funktionen der Antriebsbaugruppe 2 und der Anschlusskabelbaugruppe 4, auch eine sichere Funktion der Baugruppen innerhalb der Aktuatoreinheit 1 vor einer abschließenden unlösbaren Verbindung der Baugruppen miteinander geprüft und sichergestellt werden kann. Dadurch können sowohl die Antriebsbaugruppe 2 als auch die Anschlusskabelbaugruppe 4 separat hergestellt werden, wodurch bei der Herstellung der Antriebsbaugruppe 2 keine Anschlusskabel 18 beachtet werden müssen. Neben dem Schutz der elektrischen Kontaktierung in der Antriebsbaugruppe 2, werden so auch die mechanischen Belastungsrisiken an den elektrischen Verbindungsstellen der Antriebsbaugruppe 2 reduziert. Darüber hinaus ermöglicht es die separate Herstellung der Antriebsbaugruppe 2 und der Anschlusskabelbaugruppe 4, diverse Herstellungsschritte außerhalb von Reinräumen zur keimfreien Produktion von Implantaten vorzunehmen und die Antriebsbaugruppe 2 und die Anschlusskabelbaugruppe 4 vor dem abschließenden Verfahrensschritt der unlösbaren Verbindung separat auf ihre Funktion und die Dichtigkeit überprüfen zu können.

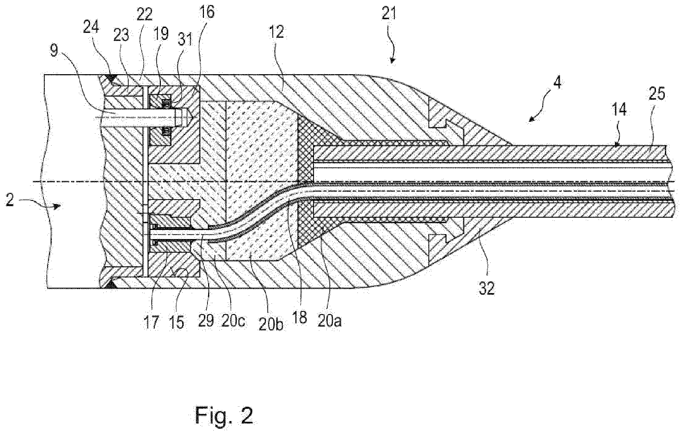

Die geschnittene Seitenansicht in Fig. 2 zeigt die unlösbar miteinander verbundene Antriebsbaugruppe 2 und Anschlusskabelbaugruppe 4 der Aktuatoreinheit 1 eines Implantats. Die in Fig. 1 in einem voneinander getrennten Montagezustand gezeigten Baugruppen 2, 4 der Aktuatoreinheit 1 sind hier miteinander verbunden, wobei die aus dem Motorgehäuse 11 der Antriebsbaugruppe 2 axial vorstehenden Kontaktstifte 9 in die Kontaktbuchsen 19 der elektrisch isolierenden Aufnahme 16 der Anschlusskabelbaugruppe 4 hineinragen und elektrisch verbunden sind. Der Anschlussflansch 22 der Anschlussöffnung 15 der Anschlusskabelbaugruppe 4 überdeckt vollständig die Anschlussstufe 23 der Antriebsbaugruppe 2. Im Kontaktbereich des Motorgehäuses 11 der Antriebsbaugruppe 2 und des Steckergehäuses 12 des Steckverbinders 21 sind das Motorgehäuse 11 und das Steckergehäuse 12 miteinander verschweißt, bevorzugt mittels eines Continuous-wave Laserschweißverfahrens, um eine glatte, übergangslose Verbindung zu erzielen. Die Schweißnaht 24 zwischen dem Motorgehäuse 11 und dem Steckergehäuse 12 erstreckt sich durchgängig um den Umfang der Aktuatoreinheit 1 und ermöglicht neben der unlösbaren Verbindung zwischen der Antriebsbaugruppe 2 und der Anschlusskabelbaugruppe 4 eine zusätzliche hermetische Abdichtung gegenüber Körperflüssigkeiten, denen die Aktuatoreinheit 1 eines Implantats im Körper eines Patienten ausgesetzt ist.

Aus der Explosionsdarstellung der Anschlusskabelbaugruppe 4 in Fig.3 ist neben dem Aufbau des Steckverbinders 21 auch dessen Montage zu erkennen. Der Kabelstrang 14 erstreckt sich durch die Kabelöffnung 13 am schmalen Ende des Steckergehäuses 12, in das Steckergehäuse 12 hinein und gibt an seinem vorstehenden Ende die Anschlusskabel 18 frei, die aus dem Kabelmantel 25 des Kabelstrangs 14 vorstehen. Die Anschlusskabel 18 weisen blanke Kabelenden 29 oder alternativ auf die Kabelenden aufgebrachte Crimp-Hülsen auf, die durch die elektrisch isolierende Aufnahme 16 und die Ausnehmung 27 für die Kontaktelemente 28 bis in die Kabelbuchsen 17 der Kontaktelemente 28 hindurch erstrecken und dort mit den Kabelbuchsen 17 elektrisch verbunden sind. Die Kontaktbuchsen 19 weisen eine innenliegende Nut (nicht gezeigt) auf, in der eine elektrisch leitende, schräg gewickelte Feder 31 eingelegt ist, die einen sicheren elektrischen Kontakt zu den Kontaktstiften 9 erzeugt. Die Kabelbuchsen 17 und die Kontaktbuchsen 19 sind vorzugsweise Bestandteil des elektrischen Kontaktelements 28 und einteilig mit diesem ausgeführt. Die Kontaktbuchse 19 ist zumindest im Bereich der Stromübertragung vorzugsweise mit einer Beschichtung, die mehrlagig sein kann und deren äußerste Schicht aus Gold besteht, ausgeführt. Weiter ist die Kontaktbuchse 19 derart ausgestaltet, dass die schräggewickelte Feder 31 radial und axial vorgespannt ist. Damit die schräggewickelte Feder 31 einfach in die Kontaktbuchse 19 eingelegt werden kann und nicht aufwändig in die Nut gedrückt werden muss, entsteht die, für den axial vorgespannten Zustand der schräggewickelten Feder 31 optimale Nutbreite erst, wenn das Kontaktelement 28 mit der Kontaktbuchse 19 flächenbündig fest mit der elektrisch isolierenden Aufnahme 16 verbunden ist. Die Kontaktelemente 28 sind fest in den Ausnehmungen 27 der elektrisch isolierenden Aufnahme 16 verankert, beispielsweise durch Einkleben, Einpressen, thermisches Fügen oder einem anderen Fügeverfahren bzw. einer Kombination von unterschiedlichen Fügeverfahren. In dem zusammengebauten Zustand der Anschlusskabelbaugruppe 4, werden die sich innerhalb des Steckergehäuses 12 ergebenden Hohlräume mittels einer Vergussmasse 20 zur Abdichtung und Fixierung des Kabelstrangs 14 sowie der Anschlusskabel 18 vergossen. Dabei kann die Vergussmasse 20 durch eine mittige Bohrung 30 in der elektrisch isolierenden Aufnahme 16 in die Hohlräume im Steckverbinder 21 eingebracht werden. Schließlich wird auch die Bohrung 30 in der elektrisch isolierenden Aufnahme 16 mit der Vergussmasse 20 gefüllt, um eine möglichst gute Abdichtung und geringe Oberfläche der Anschlusskabelbaugruppe 4 zu erzielen.

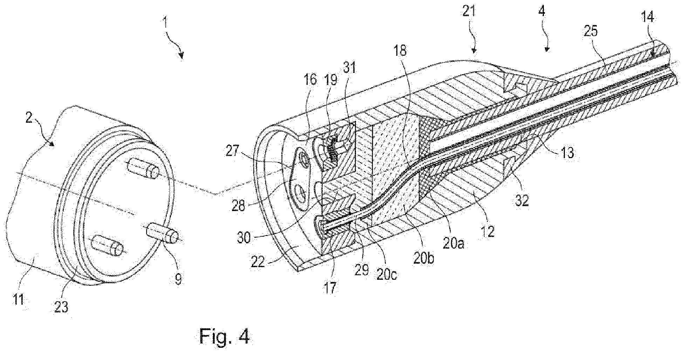

Fig. 4 zeigt eine perspektivische, teilweise geschnittene Darstellung, der noch voneinander getrennten Antriebsgruppe 2 und Anschlusskabelbaugruppe 4 der Aktuatoreinheit 1 aus Fig. 1. An der Antriebsbaugruppe 2 sind neben dem Motorgehäuse 11 und der Anschlussstufe 23 auch die stirnseitigen herausragenden Kontaktenden der mit einer Vergussmasse 10 vergossenen Kontaktstifte 9 zu erkennen. An der Anschlusskabelbaugruppe 4 ist neben dem Anschlussflansch 22 im Steckergehäuse 12 des Steckverbinders 21 sehr gut die in den Ausnehmungen 27 der elektrisch isolierenden Aufnahme 16 angeordneten Kontaktelemente 28 mit den in den Kabelbuchsen 17 elektrisch verbundenen Kabelenden 29 der Anschlusskabel 18, sowie die Kontaktbuchsen 19, welche vorzugsweise einteilig Bestandteil der Kontaktelemente 28 sind, mit der in der Nut angeordneten Feder 31 zu erkennen. An dem Ende der Kabelöffnung 13 des Steckergehäuses 12 ist eine Endkappe 32 vorgesehen, die bevorzugt aus einem elastischen Material ausgebildet ist und eine gute Abdichtung gegenüber dem Kabelmantel 25 des Kabelstrangs 14 bildet. Alternativ kann die Endkappe auch als Kabelumspritzung oder Kabelverguss ausgeführt sein, um einen sauberen, nahtlosen und strömungsoptimalen Übergang von dem metallischem Steckergehäuse 12 auf den Kabelmantel 25 zu gewährleisten, wodurch sich zusätzlich auch eine gewisse Zugentlastung und ein Knickschutz ergibt.

In der Schnittdarstellung der Anschlusskabelbaugruppe 4 in Fig. 4 ist darüber hinaus zu erkennen, dass zum Vergießen der Hohlräume im Steckergehäuse 12 unterschiedliche Vergussmassen 20a, 20b und 20c verwendet worden sind, bevorzugt biokompatible Silikon-Vergussmassen und/oder Epoxidharz-Vergussmassen. Zur besseren Füllung des Spalts zum Kabelmantel 25 wird vor dem Einschieben der elektrisch isolierenden Aufnahme 16 in die Anschlussöffnung 15 des Steckergehäuses 12, ein Teil der Vergussmasse 20, vorzugsweise eine biokompatible Silikon- oder Epoxidharzvergussmasse in das Steckergehäuse 12 eingebracht, sodass beim Einschieben der isolierenden Aufnahme 16 in die Anschlussöffnung 15 die Vergussmassenschicht 20a in den schmalen, kreisförmigen Spalt um den Kabelmantel 25 gedrückt wird. Danach wird eine mittlere Vergussmassenschicht 20b durch die Bohrung 30 in der isolierenden Aufnahme 16 in den Hohlraum im Steckergehäuse 12 eingebracht, die sich um die Anschlusskabel 18 legt, während eine dritte Vergussmassenschicht 20c anschließend über die Bohrung 30 in den oberen Teil des Hohlraums im Steckergehäuse 12 eingebracht wird, die die blanken Kabelenden 29 isoliert, eine mögliche Öffnung zwischen verdrillten Einzelleitern und der Kabelisolation dicht verschließt und sicher die elektrisch isolierende Aufnahme 16 in der Anschlussöffnung 15 fixiert. Die Vergussmassenschichten 20a und/oder 20b sind hierbei bevorzugt mit einer zähflüssigen Vergussmasse ausgeführt, damit sie bei der Montage der Anschlusskabelbaugruppe 4 nicht "auslaufen", während die Vergussmassenschicht 20c bevorzugt mit einer niederviskosen, dünnflüssigen Vergussmasse ausgeführt ist.

Der Aufbau und die Wirkungsweise der verschiedenen Vergussmassenschichten 20a, 20b, 20c ist noch einmal sehr deutlich in der teilweise geschnittenen Ansicht der Aktuatoreinheit 1 aus Fig. 2 zu erkennen. Dabei wird deutlich, dass die obere Schicht der Vergussmasse 20c im Bereich der blanken Kabelenden 29 zwischen den Anschlusskabeln 18 und den Kabelbuchsen 17 eine sichere Fixierung der elektrisch isolierenden Aufnahme 16 in der Anschlussöffnung 15 des Steckergehäuses 12 ermöglicht. Weiter ist in dieser Darstellung auch gut der am Steckergehäuse 12 der Anschlusskabelbaugruppe 4 ausgebildete Anschlussflansch 22 und die Anschlussstufe 23 des Motorgehäuses 11 der Antriebsbaugruppe 2 zu erkennen.

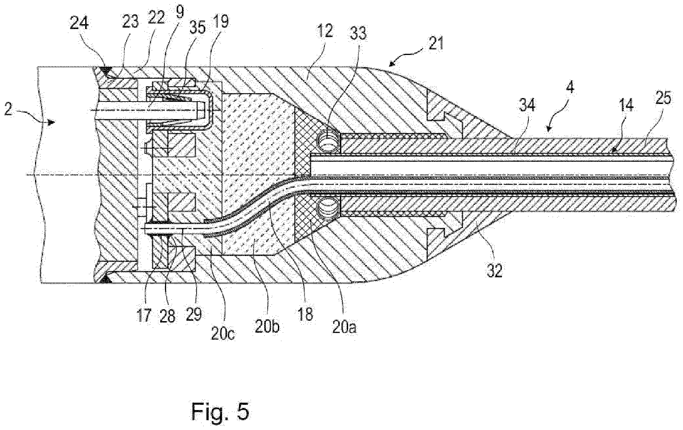

Fig. 5 zeigt eine weitere Ausführungsform der implantierbaren Anschlusskabelbaugruppe 4 für eine Aktuatoreinheit 1 aus Fig. 2 und im Gegensatz zu der vorher gezeigten Ausführungsform ist hier am Ende des Kabelstrangs 14 innerhalb des Steckergehäuses 12 der Kabelmantel 25 entfernt und mittels einer zusätzlichen schräg gewickelten elektrisch leitenden Feder 33 eine elektrische Verbindung zwischen dem Kabelschirm 34 und dem elektrisch leitenden Steckergehäuse 12 des Steckverbinders 21 hergestellt. Die Feder 33 kann in einer bevorzugten Ausführung axial vorgespannt sein. Die elektrisch leitende Verbindung zwischen dem Kabelschirm 34 und dem Steckergehäuse 12 mittels der schräg gewickelten Feder 33 ermöglicht einen Verzicht auf einen zusätzlichen Erdungsanschluss oder eines zusätzlichen Erdungskabels zwischen dem Kabelschirm 34 des Kabelstrangs 14 und dem Steckergehäuse 12. Darüber hinaus sind die Kontaktelemente 28 zwischen den Kabelbuchsen 17 und den Kontaktbuchsen 19 direkt auf der Rückseite der elektrisch isolierenden Aufnahme 16 aufgebracht und als eine elektrische Leiterplatte ausgeführt, und durch die obere Schicht der Vergussmasse 20c elektrisch isoliert. Die Kontaktierung zwischen den aus der Antriebsbaugruppe 2 vorstehenden Kontaktstiften 9 und der Kontaktbuchse 19 erfolgt hier alternativ über biegsame Fingerelemente 35. Die Kontaktierung mittels einer schräg gewickelten Feder, wie in den Fig. 1 bis 4 dargestellt, ist hier aber auch möglich.

### Bezugszeichenliste

- 1: Aktuatoreinheit
- 2: Antriebsbaugruppe
- 4: Anschlusskabelbaugruppe
- 9: Kontaktstifte
- 10: Vergussmasse
- 11: Motorgehäuse
- 12: Steckergehäuse
- 13: Kabelöffnung
- 14: Kabelstrang
- 15: Anschlussöffnung
- 16: Isolierende Aufnahme
- 17: Kabelbuchsen
- 18: Anschlusskabel
- 19: Kontaktbuchsen
- 20: Vergussmasse
- 20a: Vergussmassenschicht
- 20b: Vergussmassenschicht
- 20c: Vergussmassenschicht
- 21: Steckverbinder
- 22: Anschlussflansch
- 23: Anschlussstufe
- 24: Schweißnaht
- 25: Kabelmantel
- 27: Ausnehmung
- 28: Kontaktelemente
- 29: Kabelenden
- 30: Bohrung
- 31: Feder
- 32: Endkappe
- 33: Feder
- 34: Kabelschirm
- 35: Biegsame Fingerelemente

## Patentansprüche

1. Implantierbare Anschlusskabelbaugruppe (4) für eine Aktuatoreinheit (1) eines Implantats mit einem Steckverbinder (21) und einem in dem Steckverbinder (21) endenden Kabelstrang (14) mit mindestens zwei Anschlusskabeln (18), wobei der Steckverbinder (21) ein Steckergehäuse (12) und mindestens zwei mit jeweils einem Anschlusskabel (18) verbundene Kontaktelemente (28) aufweist, wobei die Hohlräume zwischen dem Steckergehäuse (12), den Kontaktelementen (28) und dem Kabelstrang (14) mit einer Vergussmasse (20) vergossen sind, und wobei die mindestens zwei Kontaktelemente (28) in dem Steckergehäuse (12) fixiert sind und jeweils eine Kabelaufnahme zur elektrischen Verbindung mit dem Anschlusskabel (18) des Kabelstrangs und eine separate Verbindungsschnittstelle zur elektrischen Verbindung mit der Antriebsbaugruppe (2) der Aktuatoreinheit (1) aufweisen, wobei die separaten Verbindungsschnittstellen der mindestens zwei Kontaktelemente (28) als Kontaktbuchsen (19) zur Aufnahme von Kontaktstiften (9) ausgebildet sind,
**dadurch gekennzeichnet, dass** die Kontaktbuchsen (19) zur Aufnahme von Kontaktstiften (9) eine Nut aufweisen, in der eine elektrisch leitende, schräg gewickelte Feder (31) angeordnet ist, um einen elektrischen Kontakt mit den Kontaktstiften (9) auszubilden.

2. Implantierbare Anschlusskabelbaugruppe (4) nach Anspruch 1, **dadurch gekennzeichnet, dass** eine elektrisch isolierende Aufnahme (16) vorgesehen ist, in der die Kontaktelemente (28) fixiert sind, wobei die elektrisch isolierende Aufnahme (16) in dem Steckergehäuse (12) aufgenommen ist.

3. Implantierbare Anschlusskabelbaugruppe (4) nach Anspruch 2, **dadurch gekennzeichnet, dass** die elektrisch isolierende Aufnahme (16) eine Bohrung (30) aufweist, bevorzugt eine mittig angeordnete Bohrung (30), wobei die Vergussmasse (20) zum Vergießen der Hohlräume durch die Bohrung (30) in das Steckergehäuse (12) eingebracht ist.

4. Implantierbare Anschlusskabelbaugruppe (4) nach einem der Ansprüche 1 bis 3, **dadurch gekennzeichnet, dass** die Vergussmasse (20) eine biokompatible Silikon-Vergussmasse und/oder eine biokompatible Epoxidharz-Vergussmasse ist, wobei die Vergussmasse bevorzugt aus mindestens zwei Bereichen unterschiedlicher Viskosität besteht, wobei mindestens ein Bereich aus einer dünnflüssigen Vergussmasse und mindestens ein Bereich aus einer zähflüssigen Vergussmasse besteht.

5. Implantierbare Anschlusskabelbaugruppe (4) nach einem der Ansprüche 1 bis 4, **dadurch gekennzeichnet, dass** die Kontaktelemente (28) vergoldet oder aus einer korrosionsbeständigen Metalllegierung ausgebildet sind.

6. Implantierbare Anschlusskabelbaugruppe (4) nach einem der Ansprüche 1 bis 5, **dadurch gekennzeichnet, dass** das Steckergehäuse (12) aus einem schweißbaren biokompatiblen Metall ausgebildet ist, bevorzugt aus einem korrosionsbeständigen, schweißbaren Metall.

7. Implantierbare Anschlusskabelbaugruppe (4) nach einem der Ansprüche 1 bis 6, **dadurch gekennzeichnet, dass** in dem Hohlraum zwischen dem Kabelstrang (14) und dem Steckergehäuse (12) ein Erdungskontakt vorgesehen ist, bevorzugt ein schräggewickelter Federkontakt.

8. Implantierbare Anschlusskabelbaugruppe nach einem der Ansprüche 1 bis 7, **dadurch gekennzeichnet, dass** die implantierbare Anschlusskabelbaugruppe (4) mit einer Antriebsbaugruppe (2) mit Elektromotor zu einer Aktuatoreinheit (1) eines Implantats verbindbar ist.

9. Aktuatoreinheit (1) eines Implantats aus einer separat hergestellten Antriebsbaugruppe (2) mit Elektromotor und einer separat hergestellten implantierbaren Anschlusskabelbaugruppe (4) nach einem der Ansprüche 1 bis 8, wobei die Antriebsbaugruppe (2) und die implantierbare Anschlusskabelbaugruppe (4) unlösbar miteinander verbunden sind, insbesondere glatt und übergangslos laserverschweißt sind.

10. Verfahren zum Herstellen einer Aktuatoreinheit (1) eines Implantats mit einer Antriebsbaugruppe (2) mit Elektromotor und einer implantierbaren Anschlusskabelbaugruppe (4) nach einem der Ansprüche 1 bis 8, wobei das Verfahren umfasst:
separates Herstellen der Antriebsbaugruppe (2), wobei die Antriebsbaugruppe (2) elektrische Kontakte zum elektrischen Verbinden mit der Anschlusskabelbaugruppe (4) aufweist;
separates Herstellen der implantierbaren Anschlusskabelbaugruppe (4) mit einem Steckverbinder (21) und einem in dem Steckverbinder (21) endenden Kabelstrang (14), wobei der Steckverbinder (21) mindestens zwei Kontakteelemente (28) zum elektrischen Verbinden mit den elektrischen Kontakten der Antriebsbaugruppe (2) aufweist, wobei die mindestens zwei Kontaktelemente (28) jeweils eine Kabelaufnahme zur elektrischen Verbindung mit einem Anschlusskabel (18) des Kabelstrangs und eine separate Verbindungsschnittstelle zur elektrischen Verbindung mit der Antriebsbaugruppe (2) der Aktuatoreinheit (1) aufweisen, und wobei die separaten Verbindungsschnittstellen der mindestens zwei Kontaktelemente (28) als Kontaktbuchsen (19) zur Aufnahme von Kontaktstiften (9) ausgebildet sind;
Anordnen einer elektrisch leitenden, schräg gewickelten Feder (31) in einer Nut in den Kontaktbuchsen (19), um einen elektrischen Kontakt mit den Kontaktstiften (9) auszubilden;
Fixieren der mindestens zwei Kontaktelemente (28) in dem Steckergehäuse (12) des Steckverbinders (21);
lösbares Verbinden der Antriebsbaugruppe (2) und der implantierbaren Anschlusskabelbaugruppe (4) während der Montage der Aktuatoreinheit (1) und
anschließendes unlösbares Verbinden der Antriebsbaugruppe (2) und der implantierbaren Anschlusskabelbaugruppe (4).

11. Verfahren zum Herstellen einer Aktuatoreinheit (1) eines Implantats nach Anspruch 10, wobei die separat hergestellte Antriebsbaugruppe (2) und die separat hergestellte implantierbare Anschlusskabelbaugruppe (4) hermetisch gegen Flüssigkeiten und Gase abgedichtet werden.

12. Verfahren zum Herstellen einer Aktuatoreinheit (1) eines Implantats nach Anspruch 11, wobei die separate Herstellung der implantierbaren Anschlusskabelbaugruppe (4) das Vergießen der Hohlräume des Steckverbinders (21) zwischen einem Steckergehäuse (12), dem Kabelstrang (14), den aus dem Kabelstrang (14) austretenden Anschlusskabeln und den mit den Anschlusskabeln verbundenen Kontaktelementen des Steckverbinders mit einer Vergussmasse (20) umfasst, insbesondere mit einer biokompatiblen Silikon-Vergussmasse und/oder einer biokompatiblen Epoxidharz-Vergussmasse.

13. Verfahren zum Herstellen einer Aktuatoreinheit (1) eines Implantats nach einem der Ansprüche 10 bis 12, wobei das unlösbare Verbinden der Antriebsbaugruppe (2) und der implantierbaren Anschlusskabelbaugruppe (4) in einem abschließenden Herstellungsschritt der Aktuatoreinheit (1) insbesondere mittels Schweißen und vorzugsweise mittels Continuous-wave Laserschweißen erfolgt.

## Claims

1. Implantable connection cable assembly (4) for an actuator unit (1) of an implant, having a plug connector (21) and a cable harness (14) which terminates in the plug connector (21) and has at least two connection cables (18), the plug connector (21) having a plug housing (12) and at least two contact elements (28) which each are connected to a connection cable (18), wherein cavities between the plug housing (12), the contact elements (28) and the cable harness (14) being cast with a casting compound (20), and wherein the at least two contact elements (28) are fixed in the plug housing (12) and each have a cable receptacle for electrical connection to the connecting cable (18) of the cable harness and a separate connection interface for electrical connection to the drive assembly (2) of the actuator unit (1), and wherein the separate connection interfaces of the at least two contact elements (28) are configured as contact sockets (19) for receiving contact pins (9),
**characterized in that** the contact sockets (19) for receiving contact pins (9) having a groove in which an electrically conductive, obliquely wound spring (31) is arranged in order to form an electrical contact with the contact pins (9).

2. Implantable connection cable assembly (4) according to claim 1,
**characterized in that** an electrically insulating receptacle (16) is provided in which the contact elements (28) are fixed, the electrically insulating receptacle (16) being received in the connector housing (12).

3. Implantable connection cable assembly (4) according to claim 2,
**characterized in that** the electrically insulating receptacle (16) has a bore (30), preferably a centrally arranged bore (30), wherein the casting compound (20) for casting the cavities is introduced into the connector housing (12) through the bore (30).

4. Implantable connection cable assembly (4) according to any one of claims 1 to 3, **characterized in that** the casting compound (20) is a biocompatible silicone casting compound and/or a biocompatible epoxy resin casting compound, the casting compound preferably consisting of at least two regions of different viscosity, wherein at least one region consisting of a low-viscosity casting compound and at least one region consisting of a viscous casting compound.

5. Implantable connection cable assembly (4) according to one of the claims 1 to 4, **characterized in that** the contact elements (28) are gold-plated or made from a corrosion-resistant metal alloy.

6. Implantable connection cable assembly (4) according to one of the claims 1 to 5, **characterized in that** the connector housing (12) is configured from a weldable biocompatible metal, preferably a corrosion-resistant weldable metal.

7. Implantable connection cable assembly (4) according to any of the claims 1 to 6, **characterized in that** a ground contact is provided in the cavity between the cable harness (14) and the connector housing (12), preferably an obliquely wound spring contact.

8. Implantable connection cable assembly according to any of the claims 1 to 7, **characterized in that** the implantable connection cable assembly (4) is connectable to a drive assembly (2) with electric motor to an actuator unit (1) of an implant.

9. Actuator unit (1) of an implant consisting of a separately produced drive assembly (2) having an electric motor and a separately produced implantable connection cable assembly (4) according to one of the claims 1 to 8, wherein the drive assembly (2) and the implantable connection cable assembly (4) are non-detachably connected to one another, in particular are smoothly and seamlessly laser-welded.

10. Method of producing an actuator unit (1) of an implant comprising a drive assembly (2) with an electric motor and an implantable connection cable assembly (4) according to any one of claims 1 to 8, the method comprising:
separately producing the drive assembly (2), wherein the drive assembly (2) has electrical contacts for electrical connection of the connection cable assembly (4);
separately producing the implantable connection cable assembly (4) having a connector (21) and a cable harness (14) terminating in the connector (21), wherein the connector (21) has at least two contact elements (28) for electrical connection to the electrical contacts of the drive assembly (2), wherein the at least two contact elements (28) each having a cable receptacle for electrical connection of a connecting cable (18) of the cable harness and a separate connection interface for electrical connection to the drive assembly (2) of the actuator unit (1), and wherein the separate connection interfaces of the at least two contact elements (28) are configured as contact sockets (19) for receiving contact pins (9);
arranging an electrically conductive, obliquely wound spring (31) in a groove in the contact sockets (19) to form an electrical contact with the contact pins (9);
fixing the at least two contact elements (28) in the connector housing (12) of the connector (21);
releasably connecting the actuator assembly (2) and the implantable connecting cable assembly (4) during mounting of the actuator unit (1); and
subsequently non-releasably connecting of the actuator assembly (2) and the implantable connection cable assembly (4).

11. Method of producing an actuator assembly (1) of an implant according to claim 10, wherein the separately produced drive assembly (2) and the separately produced implantable connection cable assembly (4) are hermetically sealed against liquids and gases.

12. Method of producing an actuator assembly (1) of an implant according to claim 11, wherein the separately produced implantable connection cable assembly (4) comprises casting the cavities of the connector (21) between a connector housing (12), the cable harness (14), the connecting cables emerging from the cable harness (14) and the contact elements of the connector connected to the connecting cables with a casting compound (20), in particular with a biocompatible silicone casting compound and/or a biocompatible epoxy resin casting compound.

13. Method for producing an actuator unit (1) of an implant according to one of the claims 10 to 12, wherein the non-detachable connection of the drive assembly (2) and the implantable connection cable assembly (4) is carried out in a final producing step of the actuator unit (1), in particular by means of welding and preferably by means of continuous-wave laser welding.

## Revendications

1. Ensemble de câbles de connexion implantable (4) pour une unité d'actionneur (1) d'un implant comportant un connecteur enfichable (21) et un faisceau de câbles (14) se terminant dans le connecteur enfichable (21) avec au moins deux câbles de connexion (18), dans lequel le connecteur enfichable (21) comporte un boîtier de fiche (12) et au moins deux éléments de contact (28) reliés chacun à un câble de connexion (18), dans lequel les cavités entre le boîtier de fiche (12), les éléments de contact (28) et le faisceau de câbles (14) est moulé avec un composé de moulage (20), et dans lequel lesdits au moins deux éléments de contact (28) sont fixés dans le boîtier de fiche (12) et comportent chacun un support de câble pour la connexion électrique au câble de connexion (18) du faisceau de câbles et une interface de connexion séparée pour le raccordement électrique au groupe d'entraînement (2) de l'unité d'actionneur (1), dans lequel les interfaces de connexion séparées desdits au moins deux éléments de contact (28) sont constituées sous forme de douilles de contact (19) pour recevoir des broches de contact (9),
**caractérisé en ce que** les douilles de contact (19) destinées à recevoir des broches de contact (9) comportent une rainure dans laquelle est enroulé en oblique un ressort électriquement conducteur (31) pour établir un contact électrique avec les broches de contact (9).

2. Ensemble de câbles de connexion implantable (4) selon la revendication 1, **caractérisé en ce qu'**une prise électriquement isolante (16) dans laquelle sont fixés les éléments de contact (28) est pourvue, dans lequel la prise électriquement isolante (16) est logée dans le boîtier de fiche (12).

3. Ensemble de câbles de connexion implantable (4) selon la revendication 2, **caractérisé en ce que** la prise électriquement isolante (16) comporte un trou (30), de préférence un trou (30) agencé au centre, dans lequel le composé de moulage (20) pour le moulage des cavités est apporté à travers le trou (30) dans le boîtier de fiche (12) .

4. Ensemble de câbles de connexion implantable (4) selon l'une des revendications 1 à 3, **caractérisé en ce que** le composé de moulage (20) est un composé de moulage à base de silicone biocompatible et/ou un composé de moulage à base de résine époxy biocompatible, dans lequel le composé de moulage est de préférence constituée d'au moins deux parties de viscosité différente, dans lequel au moins une partie est constituée d'un composé de moulage fluide et au moins une partie est constituée d'un composé de moulage visqueux.

5. Ensemble de câbles de connexion implantable (4) selon l'une des revendications 1 à 4, **caractérisé en ce que** les éléments de contact (28) sont plaqués avec de l'or ou constitués d'un alliage métallique résistant à la corrosion.

6. Ensemble de câbles de connexion implantable (4) selon l'une des revendications 1 à 5, **caractérisé en ce que** le boîtier de fiche (12) est constitué d'un métal biocompatible soudable, de préférence d'un métal soudable résistant à la corrosion.

7. Ensemble de câbles de connexion implantable (4) selon l'une des revendications 1 à 6, **caractérisé en ce qu'**un contact de terre, de préférence un contact à ressort enroulé obliquement, est pourvu dans la cavité entre le faisceau de câbles (14) et le boîtier de fiche (12).

8. Ensemble de câbles de connexion implantable selon l'une des revendications 1 à 7, **caractérisé en ce que** l'ensemble de câbles de connexion implantable (4) peut être connecté à un ensemble d'entraînement (2) comportant un moteur électrique pour former une unité d'actionneur (1) d'un implant.

9. Unité d'actionneur (1) d'un implant constituée d'un ensemble d'entraînement (2) fabriqué séparément avec un moteur électrique et d'un ensemble de câbles de connexion implantable (4) fabriqué séparément selon l'une des revendications 1 à 8, dans lequel l'ensemble d'entraînement (2) et l'ensemble de câbles de connexion implantable (4) sont reliés entre eux de manière non détachable, en particulier par soudage laser lisse et sans soudure.

10. Procédé de fabrication d'une unité d'actionneur (1) d'un implant comportant un ensemble d'entraînement (2) avec un moteur électrique et un ensemble de câbles de connexion implantable (4) selon l'une des revendications 1 à 8, dans lequel le procédé comprend :
la fabrication séparée de l'ensemble d'entraînement (2), dans lequel l'ensemble d'entraînement (2) comporte des contacts électriques pour une connexion électrique à l'ensemble de câbles de connexion (4) ;
la fabrication séparée de l'ensemble de câbles de connexion implantable (4) avec un connecteur enfichable (21) et un faisceau de câbles (14) se terminant par le connecteur enfichable (21), dans lequel le connecteur enfichable (21) comporte au moins deux éléments de contact (28) pour la connexion électrique aux contacts électriques de l'ensemble d'entraînement (2), dans lequel lesdits au moins deux éléments de contact (28) comportent chacun un support de câble pour la connexion électrique à un câble de connexion (18) du faisceau de câbles et une interface de connexion séparée pour la connexion électrique à l'ensemble d'entraînement (2) de l'unité d'actionneur (1), et dans lequel les interfaces de connexion séparées desdits au moins deux éléments de contact (28) sont conçues sous forme de douilles de contact (19) pour recevoir des broches de contact (9) ;
l'agencement d'un ressort électriquement conducteur enroulé obliquement (31) dans une rainure présente dans les douilles de contact (19) pour constituer un contact électrique avec les broches de contact (9) ;
la fixation desdits au moins deux éléments de contact (28) dans le boîtier de fiche (12) du connecteur enfichable (21) ;
la connexion détachable de l'ensemble d'entraînement (2) et de l'ensemble de câbles de connexion implantable (4) pendant le montage de l'unité d'actionneur (1), puis la connexion non détachable de l'ensemble d'entraînement (2) et de l'ensemble de câbles de connexion implantable (4) .

11. Procédé de fabrication d'une unité d'actionneur (1) d'un implant selon la revendication 10, dans lequel l'ensemble d'entraînement (2) fabriqué séparément et l'ensemble de câbles de connexion implantable (4) fabriqué séparément sont hermétiquement fermés aux liquides et aux gaz.

12. Procédé de fabrication d'une unité d'actionneur (1) d'un implant selon la revendication 11, dans lequel la fabrication séparée de l'ensemble de câbles de connexion implantable (4) implique le coulage d'un composé de moulage (20) dans les cavités du connecteur enfichable (21) entre un boîtier de fiche (12), le faisceau de câbles (14), les câbles de connexion sortant du faisceau de câbles (14), et les éléments de contact du connecteur enfichable reliés aux câbles de connexion, en particulier avec un composé de moulage à base de silicone biocompatible et/ou un composé de moulage à base de résine époxy biocompatible.

13. Procédé de fabrication d'une unité d'actionneur (1) d'un implant selon l'une des revendications 10 à 12, dans lequel la connexion non détachable de l'ensemble d'entraînement (2) et de l'ensemble de câbles de connexion implantable (4) est effectuée dans une étape de fabrication finale de l'unité d'actionneur (1), en particulier par soudage, et de préférence par soudage laser à onde continue.
